# EUROPÄISCHE PATENTSCHRIFT

(11) **EP 0 609 698 B1**
(45) Veröffentlichungstag und Bekanntmachung des Hinweises auf die Patenterteilung: **04.06.1997**
(21) Anmeldenummer: 94100708.0
(22) Anmeldetag: 19.01.1994
(51) Int. Cl.: C07C 267/00, C08K 5/29

(54) **Verfahren zur Herstellung organischer Carbodiimide**
Process for the preparation of organic carboiimides
Procédé pour la préparation de carboiimides organiques

(30) Priorität: 01.02.1993 DE 4302697
(43) Veröffentlichungstag der Anmeldung: 10.08.1994
(73) Patentinhaber: BAYER AG, 51368 Leverkusen (DE)
(72) Erfinder: Scholl, Hans-Joachim, Dr., D-51061 Köln (DE)

(56) Entgegenhaltungen:
- DE-A- 2 552 350
- US-A- 2 853 473

## Beschreibung

Die Erfindung betrifft ein neues Verfahren zur Herstellung organischer Carbodiimide durch Phospholinoxid-katalysierte Umsetzung entsprechender organischer Isocyanate und nachfolgender CO₂-Behandlung.

Carbodiimide sind in besonders einfacher Weise mit den hierfür bei weitem wirksamsten Katalysatoren aus der Phospholinoxid-Reihe nach der grundlegenden Verfahrensweise der US-Patentschrift 2 853 473 herzustellen.

Diese hohe Katalysefähigkeit ist einerseits sehr erwünscht, um die Carbodiimidisierungsreaktion unter schonenden Bedingungen durchzuführen, andererseits ist aber bis heute kein Verfahren bekannt, das eine wirksame Abtrennung des Katalysatorsystems auf Basis Phospholinoxid aus den erfindungsgemäßen Verfahrensprodukten ohne Einschränkung gewährleistet. Eine derartige wirksame Abtrennung ist insbesondere dann von großem technischen Interesse, wem lagerstabile Carbodiimide gefordert sind, die als Hydrolyseschutzmittel selbst bei höheren Temperaturen keine störenden Nebenreaktionen verursachen. Eine einfache, destillative Abtrennung des katalyseaktiven Materials, z.B. bei der Herstellung destillierbarer Carbodiimide wie 2,6,2',6'-Tetraisopropyldiphenylcarbodiimid, hergestellt aus 2,6-Diisopropylphenylisocyanat und einem technischen Gemisch aus 1-Methyl-1-phospha-2-cyclopenten-1-oxid und 1-Methyl-1-phospha-3-cyclopenten-1-oxid als Katalysator gelingt nicht. Das Produkt bleibt "restaktiv", selbst bei Entnahme eines größeren Vorlaufs und selbst bei Mitverwendung von Trägergasen wie Stickstoff oder Argon.

Daher wird nach DAS 1 156 401 die Herstellung derartiger Carbodiimide z.B. unter KOH-Katalyse beansprucht, um die benannten, störenden Restativitäten zu verhindem. Im Vergleich zur Phospholinoxid-Katalyse sind nach diesem Verfahren aber gravierende Nachteile zu benennen: hohe Katalysatormengen, verschärfte Reaktionsbedingungen und daraus resultierend technisch mindere, gefarbte Produkte. Für nicht destillierbare (Poly-)Carbodiimide entfällt diese Verfahrensweise.

Aus DE-A 25 52 350 ist ein Verfahren zur Herstellung von lagerstabilen, katalysatorfreien Gemischen von carbodiimidisierten Mono- und/oder Polyisocyanaten bekannt, das dadurch gekennzeichnet ist, daß man Mono- und/oder Polyisocyanate in Kontakt mit hochmolekularen, in Polyisocyanaten unlöslichen Verbindungen bringt, welche die Bildung von Carbodiimidgruppen aus Isocyanatgruppen katalysieren und welche aus einer hochmolekularen Matrix und über covalente Bindungen an oder in dieser Matrix gebundenen niedermolekularen Carbodiimidisierungskatalysatoren bestehen, nach Erreichen des gewünschten Carbodiimidisierungsgrades den hochmolekularen, unlöslichen Katalysator entfernt und anschließend gegebenenfalls carbodiimidgruppenfreie Mono-und/oder Polyisocyanate hinzufügt.

Es war daher die der Erfindung zugrundeliegende Aufgabe, ein neues Verfahren zur Herstellung organischer Carbodiimide zur Verfügung zu stellen, welches die angesprochenen Mängel beheben hilft Wie überraschenderweise gefunden wurde, konnte diese Aufgabe durch die nachstehend näher beschriebene Erfindung gelöst werden.

Gegenstand der Erfindung ist ein Verfahren zur Herstellung organischer Carbodiimide durch Carbodiimidisierung von Isocyanatgruppen rnit Katalysatoren vom Phospholin-Typ, dadurch gekennzeichnet, daß am Ende der Carbodiimidisierungsreaktion das Katalysatorsystem durch Einleiten von Kohlendioxid bei Temperaturen von 100 bis 250°C im Vakuum abgetrennt wird. Die Erfindung betrifft auch die organischen Carbodiimide, die nach dem obengenannten Verfahren erhalten werden.

Als Ausgangsmaterialien für das erfindungsgemäße Verfahren können beliebige organische Isocyanate eingesetzt werden, wie in den benannten Patentschriften aufgeführt.

Besonders bevorzugt sind aromatische Mono- und Diisocyanate die nach DAS 1 156 401 in allen ortho-Stellungen zur Isocyanatgruppe substituiert sind. Hierzu gehören insbesondere 2,6-Diisopropylphenylisocyanat und 2,4,6-Trüsopropylphenyl-1,3-diisocyanat.

Das erfindungsgemäße Verfahren wird in Gegenwart der genannten hochwirksamen Katalysatoren aus der Phospholinreihe durchgeführt, z.B. eines handelsüblichen Gemisches der Phospholinoxide der Formeln:

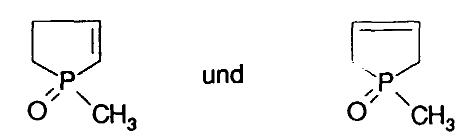

Die Menge des eingesetzten Katalysators hängt von der Qualität der Ausgangsisocyanate ab. Die jeweils notwendige Katalysatormenge läßt sich daher am einfachsten in einem Vorversuch bestimmten.

Die Carbodiimidisierungsreaktion wird gemäß Erfindung im Temperaturbereich zwischen 50 und 250°C, vorzugsweise 80 bis 200°C, durchgeführt. Die optimale Reaktionstemperatur richtet sich nach der Art der eingesetzten Ausgangsisocyanate und kann in einem einfachen Vorversuch ermittelt werden.

Die Carbodiimidisierungsreaktions wird im allgemeinen bei Erreichen eines Carbodiimidisierungsgrads (Carbodiimidisierungsgrad = Prozentsatz der carbodiimidisierten Isocyanatgruppen, bezogen auf Gesamtmenge der im Ausgangsisocyanat vorliegenden Isocyanatgruppen) von 20 bis 100, vorzugsweise 30 bis 99 %, abgebrochen. Der Carbodiimidisierungsgrad kann während des erfindigsgemäßen Verfahrens durch die Bestimmung des abnehmenden NCO-Gehalts erkannt werden, wobei ein NCO-Gehalt = 0, das Ende der Carbodiimidisierungsreaktion, einen Carbodiimidisierungsgrad von 100 bedeutet. Bei Erreichen des gewünschten Carbodiimidisierungsgrades wird in das Reaktionsgemisch unter Vakuumbedingungen bei 100 bis 250°C, vorzugsweise 150 bis 230°C, Kohlendioxid eingeleitet und die Einleitung so lange fortgesetzt, bis das erfindungsgemäße, organische Carbodiimid keinerlei Restaktivität mehr aufweist. Dies kann z.B. analytisch durch den Nachweis fehlender Spuren an Phosphor ermittelt werden, oder z.B. in einem chemischen "Schnelltest", wonach z.B. 20 Gew.-% erfindungsgemäßes Carbodiimid in 2,4-Diisocyanatotoluol 30 Min. bei 140°C gerührt wird: bei fehlender CO₂-Entwicklung, oder nicht merklich zunehmendem Anstieg des Brechungsindex, ist das erfindungsgemäße Carbodiimid ohne Restaktivität, so daß die CO₂-Einleitung beendet werden kann. Selbstverständlich lassen sich hiernach Reaktionsbedingungen und Kohlendioxid-Einleitungszeit am besten in einem Vorversuch ermitteln. Für nichtdestillierbare (Poly-)Carbodiimide ist nach Beendigung der Kohlendioxideinleitung und gegebenenfalls nachfolgender Entgasung im Vakuum das erfindungsgemäße Carbodiimid direkt verwendungsfähig.

Bei destillierbaren Carbodiimiden wird die Kohlendioxideinleitung vorzugsweise bis zur Beendigung der Entnahme eines Vorlaufs aufrechterhalten und das so von Restaktivitäten befreite "Sumpfprodukt" nachfolgend konventionell destilliert.

Die erfindungsgemäßen organischen Carbodiimide stellen wertvolle Stabilisatoren gegen hydrolytische Spaltung von Kunststoffen dar. Die folgenden Ausführungsbeispiele erläutern die Erfindung.

### Ausführungsbeispiele

### Beschreibung der Versuche

### A) Carbodiimidisierungsbeispiele

### Ausgangsprodukte

### 1) Katalysator:

Technisches Gemisch aus 1-Methyl-1-phospha-2-cyclopenten-1-oxid und 1-Methyl-1-phospha-3-cyclopenten-1-oxid

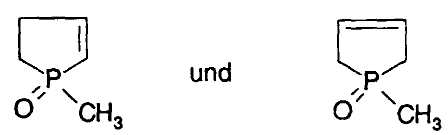

### 2) Isocyanate

### Isocyanat 1: 2,6-Diisopropylphenylisocyanat

### Isocyanat 2: 2,4,6-Triisopropylphenyl-1,3-diisocyanat

### Beispiel 1 (erfindungsgemäßes Verfahren)

812 g (4 Mol) Isocyanat 1 werden mit 0,1 g Katalysator versetzt und 27 Stunden bei 170°C gerührt. Danach ist der NCO-Gehalt von 20,7 % auf 0,5 % gefallen. Anschließend wird im Vakuum (0,4 mbar) unter CO₂-Einleitung so lange ein Vorlauf abgenommen, bis die Innentemperatur auf 170°C gestiegen ist. Die CO₂-Einleitung wird beendet und nachfolgend das erfindungsgemäße Carbodiimid überdestilliert.
(Kp 172-182°C/0,3 mbar):

| | |
|---|---|
| Vorlauf | 108 g (15 %) |
| Hauptlauf | 598 g (83 %), erfindungsgemäßes Carbodiimid |

### Beispiel 2 (Vergleichsbeispiel)

Beispiel 1 wird exakt wiederholt, die Destillation erfolgt konventionell ohne CO₂-Einleitung :

| | |
|---|---|
| Vorlauf | 110 g (15,3 %) |
| Hauptlauf | 595 g (82,6 %), Carbodiimid mit prohibitiver Restaktivität. |

### Beispiel 3 ("Schnelltest" auf Restaktivität)

Zur Prüfung auf Restaktivität werden nachfolgend unterschiedlich hergestellte Carbodiimide des Typs 2,6,2',6'-Tetraisopropyldiphenylcarbodiimid (I) getestet:

36 g (I) (0,1 Mol) werden 30 Minuten bei 140°C in 174 g (1 Mol) 2,4-Diisocyanatotoluol gerührt Bei Restaktivitäten tritt CO₂-Entwicklung ein und der Brechungsindex n_{D} steigt deutlich an. Die Daten sind in Tabelle 1 zusammengefaßt:

**Tabelle 1**

| Beispiel | CO₂ | Anstieg des Brechungsindex [n_{D}²³ °C, o-Wert, 30 Min.] | Δn_{D}²³ °C.10⁴ |
|---|---|---|---|
| 3 a | - | 1.5662 → 1.5672 | 10 |
| 3 b | + | 1.5662 → 1.5712 | 50 |
| 3 c | - | 1.5662 → 1.5672 | 10 |
| 3 d | + | 1.5662 → 1.5720 | 58 |

### Beispiel 3a

(I) nach DAS 1 156 401, bekannt unter dem Handelsnarnen Strabaxol®1 (Fa. Rhein-Chemie Rheinau GmbH). Das Produkt ist ohne Phospholinoxid-Katalyse hergestellt Beispiel 3 a ist daher der Vergleichsstandard.

### Beispiel 3 b

(I) aus Beispiel 3 a mit 0,46 ppm Katalysatorgemisch-Zusatz. Diese geringen Zusatzmengen reichen aus, deutliche Restaktivität zu provozieren.

### Beispiel 3 c

(I) aus Beispiel 1. Das erfindungsgemäße Carbodiimid ist vergleichsweise zu Beispiel 3 a ohne Restaktivität.

### Beispiel 3 d

(I) aus Beispiel 2. Das Vergleichsprodukt ist von deutlicher Restaktivität. Dieses Beispiel belegt den erfindungswesentlichen Vorteil der CO₂-Einleitung.

### Verwendungsbeispiele

### Herstellung von Elastomeren

Die Verwendungsbeispiele belegen die ausgezeichnete Stabilisatorwirkung der erfindungsgemäß hergestellten Carbodiimide in Elastomeren.

### Beispiel 4 (Vergleichsbeispiel ohne Carbodiimidzusats)

1000 g (0,5 Mol) eines entwässerten Polyesters auf Basis Adipinsäure-Ethylenglykol/Butandiol-1,4 (Gew.-Verhältnis: 1:1) mit einem zahlenmittleren Molekulargewicht von 2000 g/Mol (OH-Zahl 56) werden auf 125°C erwärmt und mit 180 g (0,86 Mol) 1,5-Naphthylendiisocyanat (NDI) intensiv verrührt. Nach 2 Min. Rührzeit wird im Vakuum entgast und nach 15 Min. das gebildete NCO-Prepolymer mit 20 g (0,22 Mol) Butandiol-1,4 verrührt. Das ca. 3,5 Min. fließfähig bleibende Reaktionsgemisch wird in eine auf 110°C vorgewärmte Form gegossen, in der es nach ca 30 Min. erstarrt.

### Beispiel 5 (Verwendungsbeispiel unter Zusatz des erfindungsgemäßen Carbo diimids aus Beispiel 1)

Beispiel 4 wird unter der Maßgabe wiederholt, daß 20 g erfindungsgemäßes Carbodiimid aus Beispiel 1 dem Polyester zugesetzt werden.

### Beispiel 6 (Vergleichsbeispiel unter Zusatz von Stabaxol®1)

Beispiel 4 wird unter der Maßgabe wiederholt, daß 20 g Stabaxol®1 dem Polyester zugesetzt werden.

Die Rezepturen und Verarbeitungsparameter sind in Tabelle 2 zusammengefaßt.

**Tabelle 2**

| | Beispiel 4 | Beispiel 5 | Beispiel 6 |
|---|---|---|---|
| Polyester (g) | 1000 | 1000 | 1000 |
| NDI (g) | 180 | 180 | 180 |
| Carbodiimid aus Beispiel 1 (g) | - | 20 | |
| Stabaxol®1 (g) | - | - | 20 |
| Butandiol-1,4 (g) | 20 | 20 | 20 |
| | | | |
| Formtemperatur (°C) | 110 | 110 | 110 |
| Gießzeit (min) | 3,5 | 1,8 | 1,8 |
| Entformungszeit (min) | | | |

In Tabelle 3 sind die physikalischen Prüfwerte aufgelistet.

**Tabelle 3**

| Eigenschaft | Prüfvorschrift | Bsp. 4 | Bsp. 5 | Bsp. 6 |
|---|---|---|---|---|
| Shore Härte A/D | DIN 53 505 | 82 | 82 | 82 |
| Stoßelastizität (%) | DIN 53 512 | 60 | 60 | 60 |
| Abriebverlust (mm³) | DIN 53 516 | 40 | 40 | 40 |
| Druckverformungsrest (%) | DIN 53 517 | 20 | 23 | 23 |
| Shore Härte A/D nach Hydrolysetest (80°C / 14 Tage) | | Probekörper durch Hydrolyse zerstört | 75 | 74 |

### Beispiel 7 (erfindungsgemäßes Verfahren zur Herstellung eines nicht destillierbaren Polycarbodiimids)

1 000 g (3,5 mol) Isocyanat 2 werden mit 0,14 g Katalysator versetzt und 4 Stunden bei 160°C gerührt. Anschließend wird im Vakuum 60 Minuten lang CO₂ eingeleitet (2 mbar, 175°C).

Der so behandelte Rückstand stellt das erfindungsgemäße Polycarbodiimid dar (NCO-Gehalt: 11,9 %), das im "Schnelltest" keine Restaktivität aufweist.

### Schnelltest-Bedingungen:

12 g Polycarbodiimid gemäß Beispiel 7 in 58 g 2,4-Diisocyanatotoluol werden bei 140°C gerührt. Selbst nach 3 Stunden bleibt der Anfangs-NCO-Wert von 41 % unverändert.

## Patentansprüche

1. Verfahren zur Herstellung organischer Carbodiimide durch Carbodiimidisierung von Isocyanatgruppen mit Katalysatoren vom Phospholin-Typ, dadurch gekennzeichnet, daß am Ende der Carbodiimidisierungsreaktion das Katalysatorsystem durch Einleiten von Kohlendioxid bei Temperaturen von 100 bis 250 °C im Vakuum abgetrennt wird.

## Claims

1. A process for the production of organic carbodiimides by carbodiimidisation of isocyanate groups using catalysts of the phospholine type, characterised in that, at the end of the carbodiimidisation reaction, the catalyst system is removed by introduction of carbon dioxide under vacuum at temperatures of from 100 to 250°C.

## Revendications

1. Procédé de préparation de carbodiimides organiques par carbodiimidation de groupes isocyanates avec des catalyseurs de type phospholine, caractérisé en ce que, à la fin de la réaction de carbodiimidation, le système catalytique est séparé sous vide par introduction de dioxyde de carbone à des températures de 100 à 250°C.
